# EUROPÄISCHE PATENTANMELDUNG

(11) **EP 1 481 631 A1**
(43) Veröffentlichungstag der Anmeldung: **01.12.2004**
(21) Anmeldenummer: 04011311.0
(22) Anmeldetag: 12.05.2004
(51) Int. Cl.: A61B 1/267

(54) **Handgriff für ein Laryngoskop**

(30) Priorität: 28.05.2003 DE 20308431 U; 14.07.2003 DE 20312563 U
(71) Anmelder: Heine Optotechnik GmbH & Co. KG, 82211 Herrsching (DE)
(72) Erfinder: Heine, Helmut M., 82211 Herrsching (DE); Fottner, Dieter, 82396 Pähl (DE); Gügel, Gerhard, 86911 Diessen (DE)
(74) Vertreter: Hano, Christian, Dipl.-Ing.

(57) **Zusammenfassung**

Der Handgriff für ein Laryngoskop umfasst ein Gehäuse (12) aus Kunststoff, an dessen oberer Stirnseite (22) eine Anschlusseinrichtung (14) zur Fixierung eines einen Lichtleiter aufweisenden Spatels und eine obere Öffnung (15) vorgesehen sind, und dessen unteres Ende offen ist, und eine das offene untere Ende des Gehäuses (12) verschließende Verschlusseinrichtung (24), die aus einer Ausschaltstellung in eine Einschaltstellung bewegbar ist, wobei die obere Öffnung (15) durch ein transparentes Material (23) verschlossen ist.

## Beschreibung

Die Erfindung betrifft einen Handgriff für ein Laryngoskop mit einem länglichen Gehäuse, an dessen oberer Stirnseite eine Anschlusseinrichtung zur Fixierung eines einen Lichtleiter aufweisenden Spatels und eine obere Öffnung vorgesehen sind, und dessen unteres Ende offen und durch eine Verschlusseinrichtung schließbar ist.

Ein solcher Handgriff ist aus dem deutschen Gebrauchsmuster 297 23 221 bekannt. Dieser Handgriff weist ein längliches zylinderförmiges Gehäuse aus Metall auf, in das eine Batterie eingesetzt ist. Am Kopfende des Gehäuses ist eine Durchgangsöffnung ausgebildet, in die eine Lampe eingesetzt ist, die durch eine Schraubenfeder nach oben vorgespannt ist, wobei die Lampe in einer Ausschaltstellung nach oben aus dem Gehäuse vorsteht. An dem Spatel ist ein Lichtleiter vorgesehen, der bei Befestigung des Spatels an der Anschlusseinrichtung gegen die Lampe drückt und diese gegen die Vorspannkraft der Feder nach unten drückt, wodurch die Lampe stromleitend mit der Batterie verbunden wird.

Laryngoskope werden zur u.a. zur Untersuchung des Kehlkopfsbereichs eines Patienten und zur Intubation verwendet. Hierbei kann es zu einer Kontamination des Spatels und des Handgriffs mit Blut, Speichel oder sonstigen Körperflüssigkeiten kommen. Um eine Kreuzkontamination von einem Patienten zum nächsten zu verhindern, ist es erforderlich, den bekannten Handgriff und den Spatel vor einem nächsten Gebrauch zu sterilisieren. Diese Sterilisierung ist jedoch sehr aufwendig.

Um eine Sterilisierung nach jedem Gebrauch zu vermeiden, ist es aus der US 4,848,558 bekannt, eine durchsichtige undurchlässige Schutzhülle über den Spatel und den Handgriff zu streifen, die nach der Untersuchung eines Patienten abgezogen und weggeworfen wird. Das Abziehen der Schutzhülle muss sehr vorsichtig vorgenommen werden, um eine Kontamination von Griff und Spateloberfläche zu verhindern.

Schließlich sind einteilige Laryngoskope für einen Einmalgebrauch bekannt, die nach einem einmaligen Einsatz komplett mit allen stromführenden Teilen entsorgt werden.

Der Erfindung liegt die Aufgabe zugrunde, mit konstruktiv einfachen Mitteln einen für einen Einmalgebrauch dienenden Handgriff für ein Laryngoskop zu schaffen, dessen nach dem Einmalgebrauch zu entsorgenden Teile kostengünstig herstellbar sind.

Diese Aufgabe wird erfindungsgemäß durch einen Handgriff mit den Merkmalen des Patentanspruchs 1 bzw. 5 gelöst. Vorteilhafte Weiterbildungen des erfindungsgemäßen Handgriffs sind Gegenstand der Patentansprüche 2 bis 4 bzw. 6.

Bei den erfindungsgemäßen Handgriffen ist das Gehäuse nach Anbringung der Verschlusseinrichtung vollständig geschlossen, so dass ein Eintritt von Speichel, Blut oder anderen Körperflüssigkeiten in den Handgriff verhindert wird. Nach dem Einsatz wird ein in das Gehäuse eingeführter Lichteinsatz entnommen, wonach der Handgriff keine stromführenden Teile mehr enthält und entsorgt wird. Die zu entsorgenden Teile, d. h. das Gehäuse, die Verschlusseinrichtung sowie ein an dem Handgriff fixierbarer Spatel sind kostengünstig herstellbar.

Ein Ausführungsbeispiel der Erfindung wird nachstehend anhand von Zeichnungen näher erläutert. Es zeigen:

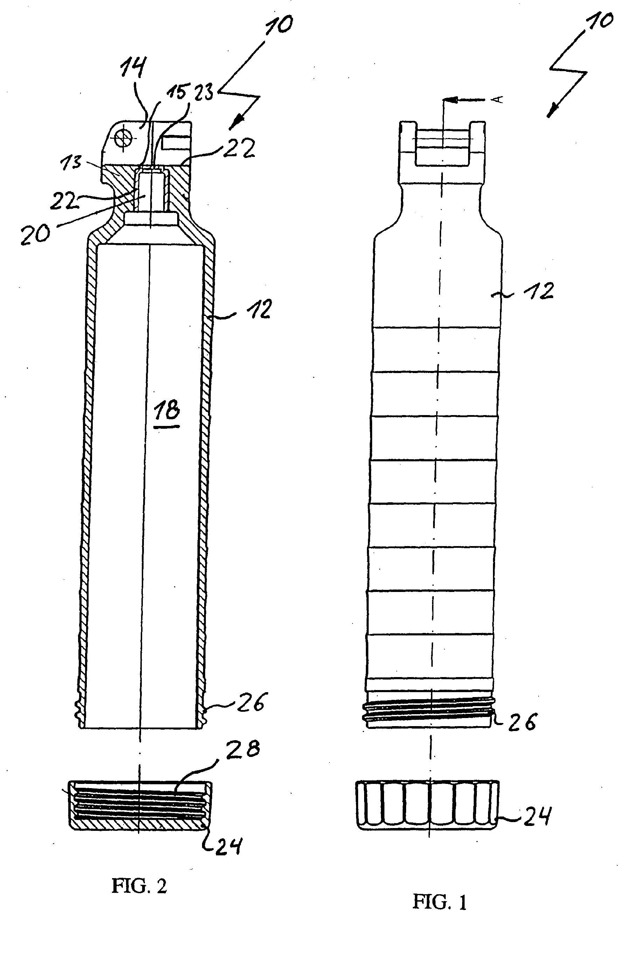

- Fig. 1: eine Seitenansicht eines Handgriffs und eines Verschlussdeckels eines Laryngoskopes;
- Fig. 2: den Schnitt A von Fig. 1;
- Fig. 3: im Querschnitt den Handgriff von Fig. 2, wobei in den Handgriff ein Leuchteinsatz eingesetzt ist und sich der Verschlussdeckel in einer Ausschaltstellung befindet;
- Fig. 4: die Ansicht von Fig. 3, wobei sich der Verschlussdeckel in einer Einschaltstellung befindet.

Der Handgriff 10 weist ein im Wesentlichen zylindrisches längliches Gehäuse 12 aus Kunststoff auf, das sich an seinem oberen Kopfabschnitt 13 verjüngt. Auf der Stirnseite 22 des Kopfabschnitts 13 ist eine Anschlusseinrichtung 14 zur Fixierung eines Spatels (nicht gezeigt) angebracht, die im Wesentlichen wie die Anschlusseinrichtung ausgebildet ist, die aus dem deutschen Gebrauchsmuster 297 23 221 bekannt ist.

Das Gehäuse 12 weist einen länglichen nach unten offenen Zylinderraum 18 auf, der sich verjüngend im Kopfabschnitt 13 in einen zylinderförmigen Kopfraum 20 übergeht, der über eine Öffnung 15 in die Stirnseite 22 mündet.

In den Zylinderraum 20 ist ein nach unten offenes, topfförmiges Einsatzstück 22 auch aus transparentem Kunststoff eingesetzt, dessen obere Stirnseite 23 bündig zur Stirnseite 22 liegt und die Öffnung 15 verschließt.

Am unteren Ende des Gehäuses 12 ist ein Außengewinde 26 ausgebildet. Auf das Außengewinde 26 ist ein topfförmiger Verschlussdeckel 24 aus Kunststoff aufschraubbar, der ein Innengewinde 28 aufweist.

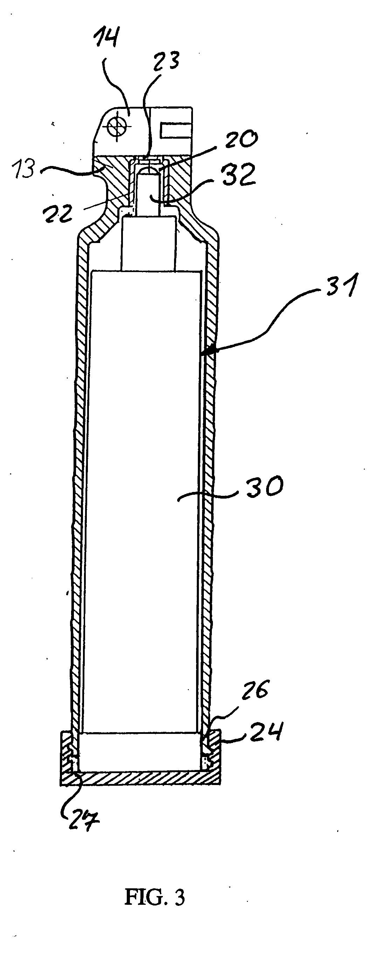

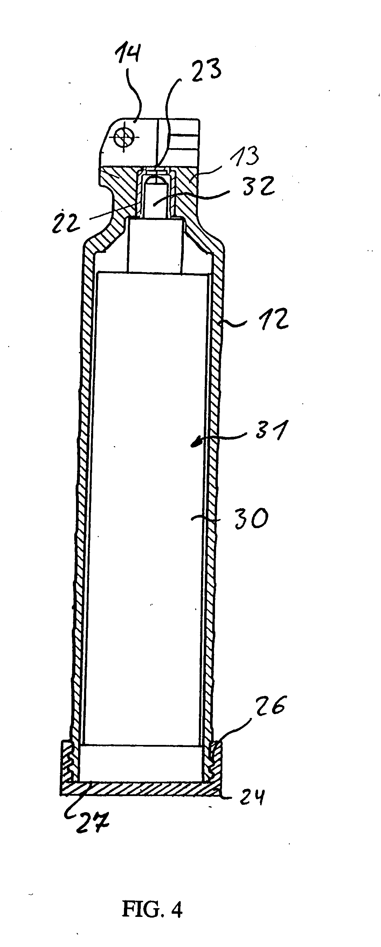

Wie es in den Fig. 3 und 4 gezeigt ist, wird für den Gebrauch in das Gehäuse 12 ein Lichteinsatz 31 eingesetzt, der einen länglichen zylinderförmigen Batteriekörper 30 aufweist. An der Oberseite des Batteriekörpers 30 ist eine Lampe 32 vorgesehen, die in das Einsatzstück 22 aus transparentem Material vorsteht. Die Lampe 32 ist nach oben durch eine Vorspanneinrichtung (nicht gezeigt) vorgespannt, und in Längsrichtung des Gehäuses 12 bzw. des Batteriekörpers 30 verschiebbar.

In der in Fig. 3 gezeigten Ausschaltstellung ist der Verschlussdeckel 24 nur teilweise auf das Gewinde 26 des Gehäuses 12 aufgeschraubt, so dass sich der Boden 27 des Verschlussdeckels 24 im Abstand zum unteren Ende des Gehäuses 12 befindet.

Die Länge des Lichteinsatzes 31 ist so ausgelegt, dass der Batteriekörper 30 in der Ausschaltstellung von Fig. 3 auf dem Boden 27 des Verschlussdeckels 24 aufliegt und sich die Lampe 32 knapp unterhalb der oberen Stirnseite 23 des Einsatzstückes 22 befindet, oder an dieser anliegt, wobei die Lampe 32 in dieser Stellung mit einem Akkumulator in dem Batteriekörper 30 nicht stromführend verbunden ist.

In der in Fig. 4 gezeigten Stellung ist der Verschlussdeckel 24 vollkommen auf das Außengewinde 26 geschraubt, so dass das untere Ende des Gehäuses 12 an dem Boden 27 anliegt. Bei dem Aufschrauben des Verschlussdeckels 24 aus der in Fig. 3 gezeigten Ausschaltstellung in die in Fig. 4 gezeigte Einschaltstellung wird der Batteriekörper 30 nach oben bewegt. Da die Lampe 32 bei dieser Bewegung an der Stirnseite 23 des Einsatzstückes 22 zum Anliegen kommt oder bereits an dieser anliegt, wird der Batteriekörper 30 relativ zu der Lampe 32 gegen die Vorspannkraft der Vorspanneinrichtung nach oben bewegt, wodurch die Lampe 32 mit dem Akkumulator in dem Batteriekörper 30 stromführend verbunden wird und leuchtet. Das Licht der Lampe 32 tritt durch die Stirnseite 23 des Einsatzstückes 22 hindurch und nach Anbringung eines Spatels in dessen Lichtleiter.

Nach dem Gebrauch wird der Verschlussdeckel 24 von dem Gehäuse 12 abgeschraubt und der Lichteinsatz 31 entnommen und in eine Ladestation eingesetzt. Das Gehäuse 12 und der Verschlussdeckel 24 werden anschließend entsorgt.

Bei einer anderen Ausführungsform des Handgriffs 10 kann das Gehäuse 12 am unteren Ende geschlossen sein und der Kopfabschnitt 13 auf das obere Ende des Gehäuses 12 aufgeschraubt werden. Der Kopfabschnitt 13 dient dann als Verschlusseinrichtung, die durch Aufschrauben aus der Ausschaltstellung in die Einschaltstellung bewegbar ist.

## Patentansprüche

1. Handgriff für ein Laryngoskop, mit
einem Gehäuse (12) aus Kunststoff, an dessen oberer Stirnseite (22) eine Anschlusseinrichtung (14) zur Fixierung eines einen Lichtleiter aufweisenden Spatels und eine obere Öffnung (15) vorgesehen sind, und dessen unteres Ende offen ist, und
einer das offene untere Ende des Gehäuses (12) verschließenden Verschlusseinrichtung (24), die aus einer Ausschaltstellung in eine Einschaltstellung bewegbar ist,
wobei die obere Öffnung (15) durch ein transparentes Material (23) verschlossen ist.

2. Handgriff nach Anspruch 1, **dadurch gekennzeichnet, dass** das Gehäuse (12) zylindrisch ist, und die Verschlusseinrichtung von einem nach oben offenen topfförmigen Verschlussdeckel (24) gebildet wird, der mit dem unteren Ende des Gehäuses (12) in Gewindeeingriff steht.

3. Handgriff nach Anspruch 1, **dadurch gekennzeichnet, dass** der Verschlussdeckel (24) ein Innengewinde (28) aufweist, das mit einem Außengewinde (26) am unteren Ende des Gehäuses (12) in Gewindeeingriff steht.

4. Handgriff nach einem der vorhergehenden Ansprüche, **dadurch gekennzeichnet, dass** in das Gehäuse (12) ein mit seinem unteren Ende an der Verschlusseinrichtung anliegender Lichteinsatz (31) eingesetzt ist, der eine Batterieeinrichtung (30) und ein oberhalb der Batterieeinrichtung (30) und unterhalb des transparenten Materials (23) angeordnetes Leuchtmittel (32) aufweist, das bezüglich der Batterieeinrichtung (30) in Längsrichtung des Gehäuses (12) verschiebbar und durch eine Vorspanneinrichtung nach oben vorgespannt ist, wobei der Lichteinsatz (31) so ausgelegt ist, dass das Leuchtmittel (32) bei einer Bewegung der Verschlusseinrichtung (24) aus der Ausschaltstellung in die Einschaltstellung an dem transparenten Material (23) anliegt oder zum Anliegen kommt und die Batterieeinrichtung (30) gegen die Vorspannkraft der Vorspanneinrichtung in Richtung des Leuchtmittels (32) bewegt wird, wodurch das Leuchtmittel (32) mit der Batterieeinrichtung (30) stromführend verbunden wird.

5. Handgriff für ein Laryngoskop, mit
einem länglichen Gehäuse (12) aus Kunststoff, dessen unteres Ende geschlossen und dessen oberes Ende offen ist,
einer das offene obere Ende des Gehäuses verschließenden Verschlusseinrichtung, die aus einer Ausschaltstellung in eine Einschaltstellung bewegbar ist,
einer an der Oberseite der Verschlusseinrichtung vorgesehen Anschlusseinrichtung zur Fixierung eines einen Lichtleiter aufweisenden Spatels,
einer in der Verschlusseinrichtung vorgesehen oberen Öffnung (15) die durch ein transparentes Material verschlossen ist.

6. Handgriff nach Anspruch 5, **dadurch gekennzeichnet, dass** in das Gehäuse ein mit seinem unteren Ende an dem unteren Ende des Gehäuses anliegender Lichteinsatz eingesetzt ist, der eine Batterieeinrichtung und ein oberhalb der Batterieeinrichtung und unterhalb des transparenten Materials angeordnetes Leuchtmittel aufweist, das bezüglich der Batterieeinrichtung in Längsrichtung des Gehäuses verschiebbar und durch eine Vorspanneinrichtung nach oben vorgespannt ist, wobei der Lichteinsatz so ausgelegt ist, dass das Leuchtmittel bei einer Bewegung der Verschlusseinrichtung aus der Ausschaltstellung in die Einschaltstellung an dem transparenten Material anliegt oder zum Anliegen kommt und die Batterieeinrichtung gegen die Vorspannkraft der Vorspanneinrichtung in Richtung des Leuchtmittels bewegt wird, wodurch das Leuchtmittel mit der Batterieeinrichtung stromführend verbunden wird.
